(19) Europäisches Patentamt
European Patent Office
Office européen des brevets

(11) **EP 3 654 930 B1**

(12) **EUROPEAN PATENT SPECIFICATION**

(45) Date of publication and mention
of the grant of the patent:
**21.05.2025 Bulletin 2025/21**

(21) Application number: **17752545.8**

(22) Date of filing: **07.08.2017**

(51) International Patent Classification (IPC):
*A61K 8/362* (2006.01)     *A61Q 11/00* (2006.01)
*A61K 8/22* (2006.01)      *A61K 8/19* (2006.01)

(52) Cooperative Patent Classification (CPC):
**A61Q 11/00; A61K 8/19; A61K 8/22; A61K 8/362**

(86) International application number:
**PCT/US2017/045661**

(87) International publication number:
**WO 2019/032082 (14.02.2019 Gazette 2019/07)**

(54) **ORAL CARE COMPOSITIONS INCLUDING CYCLIC ANHYDRIDES**

MUNDPFLEGEZUSAMMENSETZUNGEN MIT CYCLISCHEN ANHYDRIDEN

COMPOSITIONS DE SOIN BUCCAL COMPRENANT DES ANHYDRIDES CYCLIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.05.2020 Bulletin 2020/22**

(73) Proprietor: **Colgate-Palmolive Company
New York, NY 10022 (US)**

(72) Inventors:
• **HUANG, Chun
Somerset, NJ 08873 (US)**
• **XU, Guofeng
Plainsboro, NJ 08536 (US)**
• **DOGO-ISONAGIE, Cajetan
Highland Park, J 08904 (US)**

(74) Representative: **Wibbelmann, Jobst
Wuesthoff & Wuesthoff
Patentanwälte und Rechtsanwalt PartG mbB
Schweigerstrasse 2
81541 München (DE)**

(56) References cited:
EP-A1- 1 738 802       EP-A1- 2 130 528
WO-A1-2015/099643      JP-A- S52 102 439
US-A1- 2015 196 469    US-B1- 6 221 341

**Description**

**BACKGROUND**

Conventional oral care products (e.g., toothpastes, whitening gels, whitening trays, etc.) and whitening agents thereof are often utilized to whiten teeth. For example, conventional mouthwashes including hydrogen peroxide are often utilized to oxidize chromophores bound to surfaces of teeth to thereby whiten the teeth. While mouthwashes including hydrogen peroxide have proven to be effective for whitening teeth, different chromophores on the surfaces are often oxidized at varying rates and/or via varying mechanisms. Accordingly, mouthwashes including a single whitening agent (e.g., hydrogen peroxide) may require relatively longer periods of treatment to appreciably whiten the teeth.

In view of the foregoing, oral care products incorporating hydrogen peroxide often include an additional whitening agent to facilitate the oxidation of the different chromophores to thereby shorten the periods of treatment. While the oral care products incorporating a variety of whitening agents have demonstrated increased efficacy in whitening teeth, there is a desire to utilize whitening agents having relatively increased reactivity to thereby further reduce the periods of treatment. The whitening agents having relatively increased reactivity, however, are often unstable and subject to degradation. For example, the whitening agents having relatively increased reactivity often react with other components of the oral care products and/or degrade, thereby reducing the effectiveness thereof.

What is needed, then, are improved oral care products and whitening compositions thereof, and methods for generating whitening agents from the whitening compositions.

EP 2130528 A1 discloses a tooth bleaching material comprising hydrogen peroxide and/or a compound generating hydrogen peroxide in an aqueous solution, and a dicarboxylic anhydride. US 2015/196469 A1 discloses a mouthwash comprising hydrogen peroxide and a polymer-based cyclic anhydride.

**BRIEF SUMMARY**

The present invention concerns a mouthwash whitening composition, comprising an orally acceptable vehicle, sodium carbonate, a source of hydrogen peroxide that provides hydrogen peroxide, and a cyclic anhydride that generates a peracid with the hydrogen peroxide provided by the source of hydrogen peroxide, wherein the molar ratio of the cyclic anhydride to the hydrogen peroxide provided by the source of hydrogen peroxide is from 0.8:1 to 1.3:1; wherein the orally acceptable vehicle further comprises one or more ingredient selected from antibacterial agents, anticalculus agents, humectants, surfactants and cooling agents, and wherein the cyclic anhydride is not a polymer-based cyclic anhydride.

In at least one implementation, the source of hydrogen peroxide includes at least one of hydrogen peroxide, urea peroxide, calcium peroxide, sodium perborate, a polyvinylpyrrolidone (PVP) hydrogen peroxide complex, and sodium percarbonate.

In at least one implementation, the source of hydrogen peroxide is present in the whitening composition in an amount effective to provide less than or equal to 2.0 weight % of the hydrogen peroxide, based on a total weight of the whitening composition.

In at least one implementation, the cyclic anhydride is at least one of maleic anhydride, succinic anhydride, naphthalenetetracarboxylic dianhydride, phthalic anhydride, chloromaleic anhydride, and dichloromaleic anhydride.

In at least one implementation, the cyclic anhydride is maleic anhydride.

In at least one implementation, the peracid is maleic peracid.

In at least one implementation, the cyclic anhydride is succinic anhydride.

In at least one implementation, the peracid is succinic peracid.

In at least one implementation, the peracid is generated in less than or equal to three minutes after contacting the hydrogen peroxide and the cyclic anhydride with one another.

In at least one implementation, the whitening composition further includes a fluoride ion source.

In at least one implementation, the whitening composition further includes a surfactant.

**DETAILED DESCRIPTION**

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral. It should further be appreciated that when a numerical range is disclosed herein, any numerical value falling within the range is also specifically disclosed.

The present inventors have surprisingly and unexpectedly discovered that whitening composition including a source of hydrogen peroxide and a cyclic anhydride provides a significant and unexpected increase or enhancement in whitening efficacy of teeth as compared to whitening compositions including the source of hydrogen peroxide alone.

## COMPOSITIONS

In at least one implementation, the source of hydrogen peroxide and the cyclic anhydride may be maintained separate from one another until the point of use, and at the point of use, the source of hydrogen peroxide and the cyclic anhydride may be contacted, mixed, or otherwise combined with one another. The source of hydrogen peroxide and the cyclic anhydride may be maintained in separate phases or components of the whitening composition until the point of use. For example, the source of hydrogen peroxide and the cyclic anhydride may be maintained in separation phases, such as a hydrophobic phase and a hydrophilic phase, until the point of use. In another example, the source of hydrogen peroxide and the cyclic anhydride may be maintained and stored in separate vessels or containers until the point of use. Prior to or at the point of use, the respective contents of the separate vessels or containers may be combined or otherwise contacted with one another to generate the whitening agent.

In one implementation, contacting at least a portion or component of the whitening composition with water may initiate the release of hydrogen peroxide. For example, contacting the one or more sources of hydrogen peroxide with water may initiate the release of hydrogen peroxide. In another example, contacting at least a portion of the whitening composition with water initiates the generation of the whitening agent (e.g., peracid). In yet another example, the sources of hydrogen peroxide and the cyclic anhydride be maintained in separate phases, such as hydrophobic and hydrophilic phases, and combining, mixing, or otherwise contacting the hydrophobic phase and hydrophilic phase with one another may initiate the release of hydrogen peroxide.

### Sources of Hydrogen Peroxide

The one or more sources of hydrogen peroxide may be any compound or material capable of or configured to generate hydrogen peroxide to react with the cyclic anhydride to generate the whitening agent. For example, the source of hydrogen peroxide may be or include any compound capable of or configured to provide or release hydrogen peroxide to react with the cyclic anhydride. Illustrative sources of hydrogen peroxide may be or include, but are not limited to, hydrogen peroxide, urea peroxide, calcium peroxide, sodium perborate, a cross-linked polyvinylpyrrolidone (PVP) hydrogen peroxide complex, a polyvinylpyrrolidone (PVP) hydrogen peroxide complex, sodium percarbonate, and the like, and combinations thereof. The sources of hydrogen peroxide may also be or include, but are not limited to, PEROXYDONE™ XL 10F complex, which is commercially available from Ashland Inc. of Covington, KY. In a typical implementation, the source of hydrogen peroxide includes hydrogen peroxide.

The amount or concentration of the source of hydrogen peroxide may vary widely, and may depend upon the amount of hydrogen peroxide provided or otherwise delivered by the source of hydrogen peroxide. In at least one implementation, the source of hydrogen peroxide may be present in an amount that provides from greater than 0.0 weight % to less than or equal to 10.0 weight % free hydrogen peroxide, based on a total weight of the oral care product or the whitening composition thereof. For example, the source of hydrogen peroxide may be present in an amount that provides hydrogen peroxide (e.g., free hydrogen peroxide) in an amount of from greater than 0.0 weight %, about 0.5 weight %, about 1.0 weight %, about 1.5 weight %, about 2.0 weight %, about 2.5 weight %, about 3.0 weight %, about 3.5 weight %, about 4.0 weight %, or about 4.5 weight % to about 5.5 weight %, about 6.0 weight %, about 6.5 weight %, about 7.0 weight %, about 7.5 weight %, about 8.0 weight %, about 8.5 weight %, about 9.0 weight %, about 9.5 weight %, or about 10.0 weight %, based on a total weight of the oral care product or the whitening composition thereof. In another example, the source of hydrogen peroxide may be present in an amount that provides hydrogen peroxide in an amount of from greater than 0.0 weight % to less than or equal to 10.0 weight %, about 0.5 weight % to about 9.5 weight %, about 1.0 weight % to about 9.0 weight %, about 1.5 weight % to about 8.5 weight %, about 2.0 weight % to about 8.0 weight %, about 2.5 weight % to about 7.5 weight %, about 3.0 weight % to about 7.0 weight %, about 3.5 weight % to about 6.5 weight %, about 4.0 weight % to about 6.0 weight %, or about 4.5 weight % to about 5.5 weight %. In another implementation, the source of hydrogen peroxide may be present in an amount that provides from about 0.1 weight % to less than or equal to 2.0 weight % free hydrogen peroxide, based on a total weight of the oral care product or the whitening composition thereof. For example, the source of hydrogen peroxide may be present in an amount that provides hydrogen peroxide (e.g., free hydrogen peroxide) in an amount of from about 0.1 weight %, about 0.2 weight %, about 0.3 weight %, about 0.4 weight %, about 0.5 weight %, about 0.6 weight %, about 0.7 weight %, about 0.8 weight %, about 0.9 weight %, or about 1.0 weight % to about 1.1 weight %, about 1.2 weight %, about 1.3 weight %, about 1.4 weight %, about 1.5 weight %, about 1.6 weight %, about 1.7 weight %, about 1.8 weight %, about 1.9 weight %, or about 2.0 weight %. In another example, the source of hydrogen peroxide may be present in an amount that provides hydrogen peroxide in an amount of from about 0.1 weight % to about 2.0 weight %, about 0.2 weight % to about 1.9 weight %, about 0.3 weight % to about 1.8 weight %, about 0.4 weight % to about 1.7 weight %, about 0.5 weight % to about 1.6 weight %, about 0.6 weight % to about 1.5 weight %, about 0.7 weight % to about 1.4 weight %, about 0.8 weight % to about 1.3 weight %, about 0.9 weight % to about 1.2 weight %, or about 1.0 weight % to about 1.1 weight %. In yet another example, the source of hydrogen peroxide may be present in an amount that provides hydrogen peroxide in an amount less than or equal to 2.0 weight %, less than or equal to 1.8 weight %, less than or equal to 1.6 weight %, less than or equal to 1.4 weight %, less than or equal to 1.2 weight %, less than or equal to 1.0 weight %, less than or equal to 0.8 weight %, less than or equal to 0.6 weight %, or less than or equal to 0.4 weight %. In yet another implementation, the source of hydrogen peroxide may be present in an amount that provides from greater than 0.0 weight % to less than or equal to 35.0 weight % free hydrogen peroxide, based on a total weight of the oral care product or the whitening composition thereof. For example, the source of hydrogen peroxide may be present in an amount that provides hydrogen peroxide (e.g., free hydrogen peroxide) in an amount of from greater than 0.0 weight %, about 0.5 weight %, about 1.0 weight %, about 1.5 weight %, about 2.0 weight %, about 2.5 weight %, about 3.0 weight %, about 3.5 weight %, about 4.0 weight %, about 4.5 weight %, about 5.5 weight %, about 6.0 weight %, about 6.5 weight %, about 7.0 weight %, about 7.5 weight %, about 8.0 weight %, about 8.5 weight %, about 9.0 weight %, about 9.5 weight %, or about 10.0 weight % to about 12.0 weight %, about 14.0 weight %, about 16.0 weight %, about 18.0 weight %, about 20.0 weight %, about 22.0 weight %, about 24.0 weight %, about 26.0 weight %, about 28.0 weight %, about 30.0 weight %, about 32.0 weight %, about 34.0 weight %, or less than or equal to about 35.0 weight %, based on a total weight of the oral care product or the whitening composition thereof. In a typical implementation, the source of hydrogen peroxide may be present in an amount that provides hydrogen peroxide in an amount of about 35.0 weight % or less, or about 2.5 weight % or less, or about 2.0 weight % or less.

### Cyclic Anhydride

The one or more cyclic anhydrides may be any compound or material capable of or configured to react with the hydrogen peroxide from the source of hydrogen peroxide to generate the whitening agent. Illustrative cyclic anhydrides may be or include, but are not limited to, maleic anhydride, succinic anhydride, naphthalenetetracarboxylic dianhydride, phthalic anhydride, chloromaleic anhydride, dichloromaleic anhydride, 3,4,5,6-tetrahydrophthalic anhydride, 3,4,5,6-tetrachlorophthalic anhydride, 3,4,5,6-tetrabromophthalic anhydride, and 1,4,5,6,7,7-hexachloro-(2,2,1)-5-heptene-2,3-dicarboxylic acid anhydride, and the like, and combinations thereof. It should be appreciated that any two or more of the cyclic anhydrides may be combined or mixed with one another to control or adjust one or more properties of the anhydride mixture. For example, any two or more of the cyclic anhydrides may be mixed with one another to adjust the melting point and/or the solubility of the anhydride mixture in the oral care product or the whitening composition thereof.

The molar ratio of the cyclic anhydride to the hydrogen peroxide provided by the source of hydrogen peroxide is from 0.8:1 to 1.3:1. For example, the molar ratio of the cyclic anhydride to the hydrogen peroxide provided by the source of hydrogen peroxide may be from about 0.8:1, about 0.9:1, or about 1:1 to about 1.1:1, about 1.2:1, about 1.3:1, or from, , about 0.9:1 to about 1.2:1,.

In a least one implementation, the cyclic anhydride may be provided in an amount of from about 0.1 weight % to about 5.0 weight %, based on a total weight of the oral care product or the whitening composition thereof. For example, the cyclic anhydride may be provided in an amount of from about 0.1 weight %, about 0.5 weight %, about 1.0 weight %, about 1.5 weight %, or about 2.0 weight % to about 3.0 weight %, about 3.5 weight %, about 4.0 weight %, about 4.5 weight %, or about 5.0 weight %. In another example, the cyclic anhydride may be provided in an amount of from about 0.1 weight % to about 5.0 weight %, about 0.5 weight % to about 4.5 weight %, about 1.0 weight % to about 4.0 weight %, about 1.5 weight % to about 3.5 weight %, or about 2.0 weight % to about 3.0 weight %. In another example, the cyclic anhydride may be provided in an amount greater than 0.0 weight % and less than or equal to 5.0 weight %, less than or equal to 4.5 weight %, less than or equal to 4.0 weight %, less than or equal to 3.5 weight %, less than or equal to 3.0 weight %, less than or equal to 2.5 weight %, less than or equal to 2.0 weight %, less than or equal to 1.5 weight %, less than or equal to 1.0 weight %, or less than or equal to 0.5 weight %.

In at least one implementation, the amount or concentration of the cyclic anhydride and/or the source of hydrogen peroxide may be at least partially determined by a target or desired concentration of the whitening agent to be generated in the oral care product or the whitening composition thereof. For example, in at least one implementation, the target or desired concentration of the whitening agent generated may be from about 100 ppm to about 20,000 ppm. For example, the target or desired concentration of the whitening agent generated may be from about 100 ppm, about 200 ppm, about 300 ppm, about 500 ppm, about 750 ppm, about 1,000 ppm, about 2,000 ppm, about 3,000 ppm, about 4,000 ppm, about 5,000 ppm, about 6,000 ppm, or about 7,000 ppm to about 9,000 ppm, about 10,000 ppm, about 11,000 ppm, about 12,000 ppm, about 13,000 ppm, about 14,000 ppm, about 15,000 ppm, about 16,000 ppm, about 17,000 ppm, about 18,000 ppm, about 19,000 ppm, or about 20,000 ppm. In another example, the target or desired concentration of the whitening agent generated may be from about 100 ppm to about 20,000 ppm, about 200 ppm to about 19,000 ppm, about 300 ppm to about 18,000 ppm, about 500 ppm to about 17,000 ppm, about 750 ppm to about 16,000 ppm, about 1,000 ppm to about 15,000 ppm, about 2,000 ppm to about 14,000 ppm, about 3,000 ppm to about 13,000 ppm, about 4,000 ppm to about 12,000 ppm, about 5,000 ppm to about 11,000 ppm, about 6,000 ppm to about 10,000 ppm, or about 7,000 ppm to about 9,000 ppm.

**Whitening Agent**

As discussed above, the cyclic anhydride and the hydrogen peroxide provided by the source of hydrogen peroxide may be reacted or otherwise contacted with one another to generate the whitening agent. In an exemplary implementation, the whitening agent may be a derivative of hydrogen peroxide, the molecule of which may contain one or more directly linked pairs of oxygen atoms. For example, the whitening agent may be a peroxy acid or peracid. In a typical implementation, mixing, combining, or otherwise contacting the cyclic anhydride and the hydrogen peroxide with one another may initiate the generation of the whitening agent. In a preferred implementation, the cyclic anhydride may be or include maleic anhydride and/or succinic anhydride, and the whitening agent may be or include maleic peracid and/or succinic peracid, respectively.

The amount or concentration of the whitening agent (e.g., peracid) generated from the oral care product or the whitening composition thereof may vary widely. In at least one implementation, the amount of the peracid generated may be from about 0.1 ppm to about 20,000 ppm based on a total weight of an oral care product (*e.g.*, dentifrice, whitening gel, *etc.*) or the whitening composition thereof. For example, the amount of the peracid generated may be from about 0.1 ppm, about 0.5 ppm, about 1 ppm, about 5 ppm, about 10 ppm, about 15 ppm, about 20 ppm, about 50 ppm, about 100 ppm, about 150 ppm, about 200 ppm, about 300 ppm, about 500 ppm, about 600 ppm, about 700 ppm, about 800 ppm, or about 900 ppm to about 1,000 ppm, about 1,200 ppm, about 1,400 ppm, about 1,600 ppm, about 1,800 ppm, about 2,000 ppm, about 2,500 ppm, about 3,000 ppm, about 3,500 ppm, about 4,000 ppm, about 5,000 ppm, about 6,000 ppm, about 7,000 ppm, about 8,000 ppm, about 9,000 ppm, about 10,000 ppm, about 11,000 ppm, about 12,000 ppm, about 13,000 ppm, about 14,000 ppm, about 15,000 ppm, about 16,000 ppm, about 17,000 ppm, about 18,000 ppm, about 19,000 ppm, or about 20,000 ppm, based on a total weight of the oral care product or the whitening composition thereof. In another example, the amount of the peracid generated may be less than 0.1 ppm, less than 0.5 ppm, less than 1 ppm, less than 5 ppm, less than 10 ppm, less than 15 ppm, less than 20 ppm, less than 50 ppm, less than 100 ppm, less than 150 ppm, less than 200 ppm, less than 300 ppm, less than 500 ppm, less than 600 ppm, less than 700 ppm, less than 800 ppm, less than 900 ppm, less than 1,000 ppm, less than 1,200 ppm, less than 1,400 ppm, less than 1,600 ppm, less than 1,800 ppm, less than 2,000 ppm, less than 2,500 ppm, less than 3,000 ppm, less than 3,500 ppm, less than 4,000 ppm, less than 5,000 ppm, less than 6,000 ppm, less than 7,000 ppm, less than 8,000 ppm, less than 9,000 ppm, less than 10,000 ppm, less than 11,000 ppm, less than 12,000 ppm, less than 13,000 ppm, less than 14,000 ppm, less than 15,000 ppm, less than 16,000 ppm, less than 17,000 ppm, less than 18,000 ppm, less than 19,000, or less than 20,000, based on a total weight of the oral care product or the whitening composition thereof. In a typical implementation, the amount of the peracid generated is less than 20,000 ppm, based on a total weight of the oral care product or the whitening composition thereof.

In at least one implementation, the whitening agent may be generated within at least 3 minutes (min) from contacting the hydrogen peroxide and the cyclic anhydride with one another. In another implementation, the whitening agent may be generated within at least 3 minutes (min) from contacting the oral care product or the whitening composition thereof with water. For example, the whitening agent of the whitening composition may be generated in less than or equal to 3 min, less than or equal to 2.8 min, less than or equal to 2.6 min, less than or equal to 2.4 min, less than or equal to 2.2 min, less than or equal to 2.0 min, less than or equal to 1.8 min, less than or equal to 1.6 min, less than or equal to 1.4 min, less than or equal to 1.2 min, less than or equal to 1.0 min, less than or equal to 0.8 min, less than or equal to 0.6 min, or less than or equal to 0.4 min.

**Vehicle**

The whitening composition include or are combined with an orally acceptable vehicle to form the mouthwash. As used herein, "orally acceptable vehicle" may refer to a suitable vehicle, ingredient, or combination of ingredients, which can be used to form and/or apply the oral care composition to the surfaces of the teeth in a safe and effective manner. It should be appreciated that the orally acceptable vehicle may include materials such as, but not limited to, one or more antibacterial agents, anticalculus agents, buffers, additional abrasives, sources of peroxide (e.g., hydrogen peroxide), alkali metal bicarbonate salts, thickening materials, humectants, water, surfactants, titanium dioxide, cooling agents, coloring agents, and the like, and combinations thereof. In an exemplary implementation, the orally acceptable vehicle may include a mixture of water, glycerin, and sorbitol. In another implementation, the orally acceptable vehicle may include water and glycerin. In yet another implementation, the whitening composition is combined with an orally acceptable vehicle including a hydrophilic phase and a hydrophobic phase, and optionally a hydrotrope to form a dual-phase mouthwash or a dual-phase mouthwash composition.

*Polymers*

The oral care product or the whitening composition thereof may include one or more polymers. Illustrative polymers that may be included in the oral care product or the whitening composition thereof may include polyvinylmethyl ether maleic acid copolymers and/or polysaccharides, such as cellulose derivatives, polysaccharide gums, and the like, and combinations thereof. The cellulose derivatives may include carboxymethyl cellulose, and the polysaccharide gums may include xanthum gum or carrageenan gum.

In at least one implementation, the whitening composition may include one or more copolymers, such as a polyvinylmethylether/maleic anhydride (PVM/MA) copolymer, polymaleic anhydride, polystyrene/maleic anhydride (PS/MA) copolymer, polyethylene/maleic anhydride (PE/MA) copolymer, polyepropylene/maleic anhydride (PP/MA) copolymer, polypropylene-graft-maleic anhydride, polyethylene-graft-maleic anhydride, and polyisoprene-graft-maleic anhydride, a phosphate/acrylate copolymer, and the like, and combinations thereof. As discussed above, the polymer-based cyclic anhydrides may be provided as a cyclic anhydride. For example, the polymer-based cyclic anhydrides may be reacted with the hydrogen peroxide to generate the whitening agent in an appreciable amount (e.g., greater than 200 ppm). As set forth in claim 1, polymer-based cyclic anhydrides are not included as a cyclic anhydride. For example, the polymer-based cyclic anhydrides may not react with the hydrogen peroxide to generate the whitening agent in an appreciable amount (e.g., greater than 200 ppm). An illustrative PVM/MA copolymer may include those under the GANTREZ® brand, which is commercially available from ISP of Wayne, NJ.

*Fluoride Ion Source*

The oral care product or the whitening composition thereof may include one or more fluoride ion sources (e.g., soluble fluoride salts). A wide variety of fluoride ion-yielding materials may employed as sources of soluble fluoride. Examples of suitable fluoride ion-yielding materials may be found in U.S. Pat. No. 3,535,421 to Briner et al., U.S. Pat. No. 4,885,155 to Parran, Jr. et al., and U.S. Pat. No. 3,678,154 to Widder et al.. Illustrative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In a preferred implementation, the fluoride ion source includes sodium fluoride. The amount of the fluoride ion source present in the whitening composition may be less than 0.08 wt%. For example, the amount of the fluoride ion source present in the whitening composition may be less than 0.08 weight %, less than 0.07 weight %, less than 0.06 weight %, less than 0.05 weight %, or less than 0.04 weight %. In another implementation, the fluoride ion source is present in an amount to provide fluoride ions in a total amount of about 100 to about 20,000 ppm, about 200 to about 5,000 ppm, or about 500 to about 2,500 ppm.

*Surfactants*

The oral care product or the whitening composition thereof may include one or more surfactants. For example, the whitening composition may include one or more anionic surfactants, one or more cationic surfactants, one or more zwitterionic surfactants, one or more nonionic surfactants, and mixtures thereof. Examples of suitable surfactants may be found in U.S. Pat. No. 3,959,458 to Agricola et al., U.S. Pat. No. 3,937,807 to Haefele, and U.S. Pat. No. 4,051,234 to Gieske et al

In at least one implementation, the oral care product or the whitening composition thereof includes at least one anionic surfactant. Illustrative anionic surfactants may include, but are not limited to, water-soluble salts of higher fatty acid monoglyceride monosulfates, such as a sodium salt of a monosulfated monoglyceride of hydrogenated coconut oil fatty acids, such as sodium N-methyl N-cocoyl taurate, sodium cocomonoglyceride sulfate. Illustrative anionic surfactants may also include higher alkyl sulfates. As used herein, "higher alkyl" refers to $C_{6-30}$ alkyl. For example, in a preferred implementation the anionic surfactant is sodium lauryl sulfate. The anionic surfactants may also include higher alkyl-ether sulfates. For example, the anionic surfactants may have a formula $CH_3(CH_2)_mCH_2(OCH_2CH_2)_nOSO_3X$, where m is 6-16, n is 1-6, and X is Na or K. In an exemplary implementation, m is 10, and n is 2, 3, or 4, and X is Na or K. For example, the anionic surfactant may be sodium laureth-2 sulfate $(CH_3(CH_2)_{10}CH_2(OCH_2CH_2)_2OSO_3Na)$. In another implementation, the anionic surfactant may include higher alkyl aryl sulfonates, such as sodium dodecyl benzene sulfonate (sodium lauryl benzene sulfonate), and higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate. In an exemplary implementation, the anionic surfactant is a water soluble salt of alkyl sulfates having from 10 to 18 carbon atoms in the alkyl radical and water soluble salts of sulfonated monoglycerides of fatty acids having from 10 to 18 carbon atoms. For example, the anionic surfactant may be or include, sodium lauryl sulfate, sodium lauroyl sarcosinate, sodium coconut monoglyceride sulfonates, or the like, and mixtures thereof.

In at least one implementation, the oral care product or the whitening composition thereof may include at least one nonionic surfactant. Accordingly, the oral care product or the whitening composition thereof may include at least one anionic surfactant, at least one nonionic surfactant, or both an anionic surfactant and a nonionic surfactant. The nonionic surfactant may function as an emulsifier. Illustrative nonionic surfactants may include, but are not limited to, poloxamers or the like. For example, the nonionic surfactants may include polysorbate 20, poloxamer 407, poloxamer 338, or the like, and mixtures thereof. The nonionic surfactants may also include, but are not limited to, ethoxylated and hydrogenated ethoxylated castor oils, such as those commonly designated as PEG NN castor oil or PEG NN hydrogenated castor oil, where "NN" designates the number of ethylene oxide units polymerized onto the castor oil to form the nonionic surfactant. For example, the nonionic surfactants may be or include PEG 16, 20, 25, 30, 40, 50, 60, 80, 100, 200, and combinations thereof. In a preferred implementation, the nonionic surfactant is polysorbate 20.

The amount of any one or more of the surfactants in the oral care product or the whitening composition thereof may be from about 0.010 wt%, about 0.020 wt%, about 0.030 wt%, about 0.040 wt%, about 0.045 wt%, about 0.049 wt%, or about 0.050 wt% to about 0.051 wt%, about 0.055 wt%, about 0.060 wt%, about 0.065 wt%, about 0.070 wt%, about 0.075 wt%, about 0.080 wt%, or greater. In another example, the amount of any one or more of the surfactants in the oral care product or the whitening composition thereof may be about 0.010 wt% to about 0.090 wt%, about 0.020 wt% to about 0.080 wt%, about 0.030 wt% to about 0.070 wt%, about 0.040 wt% to about 0.060 wt%, about 0.045 wt% to about 0.055 wt%, or about 0.050 wt% to about 0.051 wt%. In yet another example, the amount of any one or more of the surfactants in the oral care product or the whitening composition thereof may be greater than 0.010 wt%, greater than 0.020 wt%, greater than 0.030 wt%, greater than 0.040 wt%, greater than 0.045 wt%, greater than 0.049 wt%, or greater than 0.050 wt%. The amount of any one or more of the surfactants in the oral care product or the whitening composition thereof may also be from about 0.10 wt%, about 0.20 wt%, about 0.30 wt%, about 0.40 wt%, about 0.45 wt%, about 0.49 wt%, or about 0.50 wt% to about 0.51 wt%, about 0.55 wt%, about 0.60 wt%, about 0.65 wt%, about 0.70 wt%, about 0.75 wt%, about 0.80 wt%, or greater. In another example, the amount of any one or more of the surfactants in the oral care product or the whitening composition thereof may be about 0.10 wt% to about 0.90 wt%, about 0.20 wt% to about 0.80 wt%, about 0.30 wt% to about 0.70 wt%, about 0.40 wt% to about 0.60 wt%, about 0.45 wt% to about 0.55 wt%, or about 0.50 wt% to about 0.51 wt%. In yet another example, the amount of any one or more of the surfactants in the oral care product or the whitening composition thereof may be greater than 0.10 wt%, greater than 0.20 wt%, greater than 0.30 wt%, greater than 0.40 wt%, greater than 0.45 wt%, greater than 0.49 wt%, or greater than 0.50 wt%.

### Flavoring Agents

The oral care product or the whitening composition thereof may also include one or more flavoring agents. Illustrative flavoring agents may include, but are not limited to, essential oils and various flavoring aldehydes, esters, alcohols, and the like. The flavoring agents may also include, but are not limited to, sweeteners, sucralose, dextrose, polydextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof (e.g., sodium saccharin), dipeptide-based intense sweeteners, cyclamates, dihydrochalcones and mixtures thereof. Examples of the essential oils include oils of spearmint, peppermint, wintergreen, sassafras, clove, sage, eucalyptus, marjoram, cinnamon, lemon, lime, grapefruit, and orange. In another example, the flavoring agents may include menthol, carvone, and anethole. In a preferred implementation, the flavoring agent includes peppermint and spearmint. In a more preferred implementation, the flavoring agent includes a Firmenich Newman Flavor. The amount of the flavoring agent in the oral care product or the whitening composition thereof may be less than 1.0 wt%, less than 0.9 wt%, less than 0.8 wt%, or less than 0.7 wt%. For example, the amount of the flavoring agent in the oral care product or the whitening composition thereof may be about 0.0 wt% to about 1.0 wt%, about 0.5 wt% to about 0.9 wt%, about 0.7 wt% to about 0.8 wt%. In a preferred implementation, the amount of the flavoring agent in the oral care product or the whitening composition thereof is about 0.75 wt% to about 0.80 wt%.

### Humectants

The oral care product or the whitening composition thereof may include one or more humectants. The humectants may be capable of or configured to reduce evaporation and lower water activity. It should be appreciated that the humectants may also be capable of imparting desirable sweetness or flavor to the oral care product or the whitening composition thereof. Illustrative humectants may include, but are not limited to polyhydric alcohols, such as glycerin, sorbitol, xylitol, propylene glycol, as well as other polyols, and mixtures thereof.

### Water

The oral care product or the whitening composition thereof may include water. Water of the oral care product or the whitening composition thereof may be deionized and free of organic impurities. Water may make up the balance of the oral care product or the whitening composition thereof. For example, the amount of water in the oral care product or the whitening composition thereof may be from about 10 wt% to 90 wt%, about 40 wt% to about 85 wt%, or about 60 wt% to about 80 wt%. In another example, the amount of water in the oral care product or the whitening composition thereof may be at least 60 wt%, at least 65 wt%, at least 70 wt%, at least 78 wt%, or at least 79 wt%. The amount of water in the oral care product or the whitening composition thereof may include free water added and water introduced with other components or materials of the oral care product or the whitening composition thereof. For example, the amount of the water in the oral care product or the whitening composition thereof may include free water and water associated with the humectants, flavoring agents, or any other component of the oral care product or the whitening composition thereof.

### Additional Components/Ingredients

The oral care product or the whitening composition thereof may optionally include one or more additional components or ingredients. For example, the oral care product or the whitening composition thereof may include one or more antimicrobial agents such as, methylisothiazolinone (MIT), sodium benzoate, potassium sorbate, and combinations thereof. In another example, the oral care product or the whitening composition thereof may include one or more antibacterial agents selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC), phenolic antiseptics, hexetidine, octenidine, sanguinarine, povidone iodine, delmopinol, salifluor, other metal ions (e.g., stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol, and other piperidino derivatives, nicin preparations, chlorite salts; and mixtures of any of the foregoing. In an exemplary implementation, the antibacterial agent includes cetylpyridinium chloride (CPC).

The oral care product or the whitening composition thereof may optionally include one or more pH modifying agents. For example, the oral care product or the whitening composition thereof may include one or more acidifying agents and/or one or more basifying agents to reduce and/or increase the pH, respectively. The oral care product or the whitening composition thereof may also include one or more buffering agents to control or modulate the pH within a predetermined or desired range. Illustrative buffering agents may include, but are not limited to, sodium bicarbonate, sodium phosphate, sodium hydroxide, sodium carbonate, sodium acid pyrophosphate, citric acid, sodium citrate, and mixtures thereof. Sodium phosphate may include, monosodium phosphate ($NaH_2PO_4$), disodium phosphate ($Na_2HPO_4$), trisodium phosphate ($Na_3PO_4$), and mixtures thereof. In a preferred implementation, the buffering agent is anhydrous sodium phosphate dibasic or disodium phosphate.

In at least one implementation, the acidifying, buffering, and/or buffering agents may be included in the oral care product or the whitening composition thereof to provide the oral care composition with a pH between 2 to 10, 2 to 8, 3 to 9, 4 to 8, 6 to 10, or 7 to 9. Additional orally acceptable pH modifying agent may be used, including without limitation carboxylic, phosphoric, and sulfonic acids, acid salts (e.g., monosodium citrate, disodium citrate, monosodium malate, etc.), alkali metal hydroxides, such as sodium hydroxide, carbonates, such as sodium carbonate, bicarbonates, sesquicarbonates, borates, silicates, phosphates (*e.g.*, monosodium phosphate, trisodium phosphate, pyrophosphate salts, *etc.*), imidazole and mixtures thereof. The one or more pH modifying agents may be optionally present in an amount effective to maintain the oral care product or the whitening composition thereof in an orally acceptable pH range. In a preferred implementation, the buffering agent includes anhydrous sodium phosphate dibasic or disodium phosphate, and phosphoric acid (*e.g.*, syrupy phosphoric acid; 85%-Food Grade).

### METHODS

In various implementations, the present disclosure provides methods to whiten surfaces of teeth in a human or animal subject in need thereof. As used herein, "animal subject" includes non-human mammals such as canines, felines, and horses. The methods may include contacting the surfaces of the teeth with the whitening composition or the whitening agent of the present disclosure. Contacting the whitening composition with the surfaces of the teeth may include brushing, flossing, irrigating, wiping, rinsing (lavage of oral cavity), foam/gel and in-tray application, masticating, spraying, painting, and the like.

In various implementations, the oral care product, or the whitening composition thereof, prepared in accordance with the present disclosure may be applied regularly to an oral surface, for example on a daily basis, at least one time daily for multiple days, or alternately every second or third day. In some implementations, the oral care product or the whitening composition thereof is applied to the oral surfaces from 1 to 3 times daily, for at least 2 weeks up to 8 weeks, from four months to three years, or more, up to a lifetime.

In some implementations, the the mouthwash thereof may be applied directly to the teeth using a delivery device, such as a pen, (*e.g.*, a COLGATE® whitening pen or a COLGATE® ACTIS™ whitening pen, Colgate-Palmolive Company, New York, NY), a liquid stick having an applicator, such as a felt tip, brush, roller ball, or non-woven pad, sufficient to effect whitening.

## EXAMPLES

The examples and other implementations described herein are exemplary and not intended to be limiting in describing the full scope of compositions and methods of this disclosure. Equivalent changes, modifications and variations of specific implementations, materials, compositions and methods may be made within the scope of the present disclosure, with substantially similar results. The scope of the invention is defined by the appended claims.

Example 1

The generation of peracid from an oral care composition, namely a mouthwash, was evaluated via HPLC and UV/Vis. Since peracid is not visible via UV-Vis, secondary compounds that are visible or absorb in the UV-Vis spectrum were derived from the generated peracid via successive oxidation reactions.

To simulate the generation of peracid, namely maleic peracid, about 100 mg (0.5 weight % to mouthwash) of a cyclic anhydride, namely maleic anhydride, and 100 mg (0.5 weight % to mouthwash) of sodium carbonate, a pH modifying agent, was combined with 20.0 g of a mouthwash containing 2 weight % hydrogen peroxide. The mouthwash mixture was agitated for 1 minute, and 1.0 mL of the agitated mixture was diluted with water to 20 mL. To derive the secondary compounds, 360 μL of the diluted mixture was then transferred to a microfuge tube containing 40 μL of 1.3 M phosphoric acid and mixed or agitated to reach a final pH of less than 3, thereby terminating the maleic anhydride ring opening reaction in generating peracid. 100 μL of mixed solution was then transferred to an HPLC container/vial containing 300 μL of water and 100 μL of a methyl tolyl sulfide (MTS) reagent, and mixed or agitated in the dark for at least 10 min, thereby reacting the peracid with the MTS reagent to produce methyl tolyl sulfoxide (MTSO) and acetic acid (AcOH). Then 400 μL of acetonitrile and 100 μL of a triphenyl phosphine (TPP) reagent was added to the solution and allowed to react in the dark for 30 min. After 30 min, 100 μL of acetonitrile was added and mixed thoroughly, and the resulting solution was analyzed via HPLC.

The calculated concentration of MTSO was then corrected for dilution (*i.e.*, during the acid quench step) and total reaction volume. It should be appreciated that the molar concentration of the peracid is equivalent to the calculated concentration of MTSO including the aforementioned corrections. The amount of the peracid generated from the hydrogen peroxide mouthwash and the maleic anhydride is summarized in Table 1.

**Table 1**

| Amount of Maleic Peracid Generated in Mouthwash | | | | | |
|---|---|---|---|---|---|
| **Weight of MA (g)** | 0 | 0.1 | 0.2 | 0.4 | 0.8 |
| **Weight of SC (g)** | 0 | 0.1 | 0.2 | 0.4 | 0.8 |
| **Weight of Mouthwash (g)** | 0 | 20.0 | 20.0 | 20.0 | 20.0 |
| **Maleic Peracid (ppm) Measured** | 0 | 5,629 | 8,938 | 15,737 | 19,930 |

As illustrated in Table 1, when more maleic anhydride (MA) and sodium carbonate (SC) were mixed with 20.0 g of a mouthwash containing 2.0 weight % hydrogen peroxide, more maleic peracid was generated. For example, about 5,600 ppm maleic peracid was formed in the when 0.1 g of MA and 0.1 g of SC were added to 20.0 g of mouthwash and over 15,000 ppm of maleic peracid was formed when 0.4 g maleic anhydride and 0.4 g of SC were added to the mouthwash. It should be appreciated that higher concentration of the peracid generated in mouthwash solution would lead to better whitening efficacy.

Example 2

The whitening efficacy of whitening compositions including 2 weight % hydrogen peroxide mouthwash with varying amounts of maleic anhydride and sodium carbonate was evaluated *in vitro.* Particularly, artificially stained bovine incisors individually mounted to resin blocks were obtained from Therametric Technologies, Inc. The artificially stained bovine teeth selected for the analysis had L* values from about 58 to about 63.

Solutions of 20 g of the 2 weight % hydrogen peroxide mouthwash (MW) with varying amounts of maleic anhydride (MA) and sodium carbonate (SC) were prepared. The bovine teeth were soaked in each of the respective solutions for 1 minute after combining the mouthwash with the maleic anhydride and sodium carbonate. Each of the bovine teeth was soaked in the solution twice to provide one treatment.

The L*, a*, and b* values were measured with a hand-held spectrophotometer after each treatment (one minute soakings for each treatment). The L*, a*, b* values after each treatment were compared to the baseline values to calculate the change in the whiteness of each of the teeth. It should be appreciated that the whiteness index (W*) is a measure of overall color change relative to pure white, and is given by formula (1), and the change in whiteness index (ΔW*) is measured by formula (2). The change in whiteness index (ΔW*) is summarized in Table 2.

$$W^* = ((L^*-100)^2 + (a^*)^2 + (b^*)^2)^{1/2} \qquad (1)$$

$$\Delta W^* = W^*_{treated} - W^*_{baseline} \qquad (2)$$

**Table 2**

| Whitening Efficacy (ΔW*) for In Vitro Treatments with Whitening Compositions | | | | | | | | | |
|---|---|---|---|---|---|---|---|---|---|
| | | **Number of Treatments** | | | | | | | |
| | | **0** | **2** | **4** | **6** | **8** | **10** | **12** | **14** |
| MW + 0%MA + 0% SC; pH = 4.5 | ΔW* | 0 | 0.607 | 1.232 | 1.250 | 1.387 | 1.506 | 2.09 | 1.731 |
| MW + 0.5% SC; pH = 6.0 | ΔW* | 0 | 0.732 | 1.387 | 1.736 | 1.412 | 1.661 | 1.784 | 2.292 |
| MW + 1%MA+ 1% SC; pH = 5.3 | ΔW* | 0 | 1.556 | 2.471 | 2.898 | 3.852 | 3.584 | 4.538 | 5.058 |
| MW + 2%MA + 2% SC; pH = 6.1 | ΔW* | 0 | 2.536 | 3.954 | 5.359 | 6.437 | 7.163 | 8.064 | 8.711 |

It was surprisingly and unexpectedly discovered, as demonstrated in Table 2, that the combination of maleic anhydride (MA) and sodium carbonate (SC) with a mouthwash (MW) containing 2.0 weight % hydrogen peroxide significantly enhanced the whitening efficacy of the whitening compositions. Particularly, the whitening composition including the maleic anhydride and the sodium carbonate provided whiter teeth at a faster rate. For example, the mouthwash including 1% MA and 1% SC had a whitening efficacy (ΔW) of about 5.1, which was about three times the whitening efficacy of the MW including the hydrogen peroxide alone with 14 treatments. The enhanced whitening efficacy (ΔW) was not attributed to the change in pH, as the whitening composition including the MW and 0.5% SC exhibited similar whitening efficacy (ΔW) as compared to the MW alone.

The present disclosure has been described with reference to exemplary implementations. Although a limited number of implementations have been shown and described, it will be appreciated by those skilled in the art that changes may be made in these implementations without departing from the principles and spirit of the preceding detailed description. It is intended that the present disclosure be construed as including all such modifications and alterations insofar as they come within the scope of the appended claims.

**Claims**

1. A mouthwash whitening composition, comprising an orally acceptable vehicle, sodium carbonate, a source of hydrogen peroxide that provides hydrogen peroxide, and a cyclic anhydride that generates a peracid with the hydrogen peroxide provided by the source of hydrogen peroxide

   wherein the molar ratio of the cyclic anhydride to the hydrogen peroxide provided by the source of hydrogen peroxide is from 0.8:1 to 1.3:1;
   wherein the orally acceptable vehicle further comprises one or more ingredient selected from antibacterial agents, anticalculus agents, humectants, surfactants and cooling agents, and

wherein the cyclic anhydride is not a polymer-based cyclic anhydride.

2. The mouthwash whitening composition of claim 1, wherein the source of hydrogen peroxide comprises at least one of hydrogen peroxide, urea peroxide, calcium peroxide, sodium perborate, a polyvinylpyrrolidone (PVP) hydrogen peroxide complex, and sodium percarbonate.

3. The mouthwash whitening composition of claim 1 or 2, wherein the source of hydrogen peroxide is present in the whitening composition in an amount effective to provide less than or equal to 2.0 weight % of the hydrogen peroxide.

4. The mouthwash whitening composition of any one of claims 1 to 3, wherein the cyclic anhydride is at least one of maleic anhydride, succinic anhydride, naphthalenetetracarboxylic dianhydride, phthalic anhydride, chloromaleic anhydride, and dichloromaleic anhydride.

5. The mouthwash whitening composition of any one of claims 1 to 4, wherein the cyclic anhydride is maleic anhydride.

6. The mouthwash whitening composition of any one of claims 1 to 5, wherein the peracid is maleic peracid.

7. The mouthwash whitening composition of any one of claims 1 to 5, wherein the cyclic anhydride is succinic anhydride.

8. The mouthwash whitening composition of claim 7, wherein the peracid is succinic peracid.

9. The mouthwash whitening composition of any one of claims 1 to 8, wherein a molar ratio of the cyclic anhydride to the hydrogen peroxide provided by the source of hydrogen peroxide is about 1:1.

10. The mouthwash whitening composition of any one of claims 1 to 9, wherein the peracid is generated in less than or equal to three minutes after contacting the hydrogen peroxide and the cyclic anhydride with one another.

11. The mouthwash whitening composition of any one of claims 1 to 10, further comprising a fluoride ion source.

12. The mouthwash whitening composition of any one of claims 1 to 11, further comprising a surfactant.

13. A method for whitening teeth of a subject, comprising contacting the mouthwash whitening composition of any one of claims 1 to 12 with surfaces of the teeth of the subject in need thereof.

14. A method for generating a whitening agent in a mouthwash whitening composition, comprising an orally acceptable vehicle, sodium carbonate, a source of hydrogen peroxide that provides hydrogen peroxide, and a cyclic anhydride that generates a peracid with the hydrogen peroxide provided by the source of hydrogen peroxide, said method comprising contacting the cyclic anhydride and hydrogen peroxide with one another, wherein the whitening agent is a peracid;

wherein the molar ratio of the cyclic anhydride to the hydrogen peroxide provided by the source of hydrogen peroxide is from 0.8:1 to 1.3:1;
wherein the orally acceptable vehicle further comprises one or more ingredient selected from antibacterial agents, anticalculus agents, humectants, surfactants and cooling agents, and
wherein the cyclic anhydride is not a polymer-based cyclic anhydride.

**Patentansprüche**

1. Mundwasser-Aufhellungszusammensetzung, umfassend einen oral akzeptablen Träger, Natriumcarbonat, eine Wasserstoffperoxidquelle, die Wasserstoffperoxid bereitstellt, und ein cyclisches Anhydrid, das mit dem von der Wasserstoffperoxidquelle bereitgestellten Wasserstoffperoxid eine Persäure erzeugt,

wobei das Molverhältnis des cyclischen Anhydrids zu dem von der Wasserstoffperoxidquelle bereitgestellten Wasserstoffperoxid 0,8:1 bis 1,3:1 beträgt;
wobei der oral akzeptable Träger weiterhin einen oder mehrere Bestandteile umfasst, ausgewählt aus antibakteriellen Mitteln, Mitteln gegen Zahnstein, Feuchthaltemitteln, Tensiden und Kühlmitteln, und
wobei das cyclische Anhydrid kein cyclisches Anhydrid auf Polymerbasis ist.

2. Mundwasser-Aufhellungszusammensetzung nach Anspruch 1, wobei die Wasserstoffperoxidquelle mindestens eines von Wasserstoffperoxid, Harnstoffperoxid, Calciumperoxid, Natriumperborat, einem Polyvinylpyrrolidon (PVP)-Wasserstoffperoxid-Komplex und Natriumpercarbonat umfasst.

3. Mundwasser-Aufhellungszusammensetzung nach Anspruch 1 oder 2, wobei die Wasserstoffperoxidquelle in der Aufhellungszusammensetzung in einer Menge vorhanden ist, die wirksam ist, um weniger als oder gleich 2,0 Gew.-% des Wasserstoffperoxids bereitzustellen.

4. Mundwasser-Aufhellungszusammensetzung nach einem der Ansprüche 1 bis 3, wobei das cyclische Anhydrid mindestens eines von Maleinsäureanhydrid, Bernsteinsäureanhydrid, Naphthalintetracarbonsäuredianhydrid, Phthalsäureanhydrid, Chlormaleinsäureanhydrid und Dichlormaleinsäureanhydrid ist.

5. Mundwasser-Aufhellungszusammensetzung nach einem der Ansprüche 1 bis 4, wobei das cyclische Anhydrid Maleinsäureanhydrid ist.

6. Mundwasser-Aufhellungszusammensetzung nach einem der Ansprüche 1 bis 5, wobei die Persäure Maleinsäurepersäure ist.

7. Mundwasser-Aufhellungszusammensetzung nach einem der Ansprüche 1 bis 5, wobei das cyclische Anhydrid Bernsteinsäureanhydrid ist.

8. Mundwasser-Aufhellungszusammensetzung nach Anspruch 7, wobei die Persäure Bernsteinsäurepersäure ist.

9. Mundwasser-Aufhellungszusammensetzung nach einem der Ansprüche 1 bis 8, wobei das Molverhältnis des cyclischen Anhydrids zu dem Wasserstoffperoxid, das durch die Wasserstoffperoxidquelle bereitgestellt wird, etwa 1:1 beträgt.

10. Mundwasser-Aufhellungszusammensetzung nach einem der Ansprüche 1 bis 9, wobei die Persäure in weniger als oder gleich drei Minuten nach dem In-Kontakt-Bringen des Wasserstoffperoxids und des cyclischen Anhydrids miteinander erzeugt wird.

11. Mundwasser-Aufhellungszusammensetzung nach einem der Ansprüche 1 bis 10, ferner umfassend eine Fluoridionenquelle.

12. Mundwasser-Aufhellungszusammensetzung nach einem der Ansprüche 1 bis 11, ferner umfassend ein Tensid.

13. Verfahren zum Aufhellen der Zähne einer Person, umfassend das In-Kontakt-Bringen der Mundwasser-Aufhellungszusammensetzung nach einem der Ansprüche 1 bis 12 mit Oberflächen der Zähne der Person, die dies benötigt.

14. Verfahren zur Erzeugung eines Aufhellungsmittels in einer Mundwasser-Aufhellungszusammensetzung, umfassend einen oral akzeptable Träger, Natriumcarbonat, eine Wasserstoffperoxidquelle, die Wasserstoffperoxid bereitstellt, und ein cyclisches Anhydrid, das mit dem von der Wasserstoffperoxidquelle bereitgestellten Wasserstoffperoxid eine Persäure erzeugt, wobei das Verfahren das In-Kontakt-Bringen des cyclischen Anhydrids und des Wasserstoffperoxids miteinander umfasst, wobei das Aufhellungsmittel eine Persäure ist;

   wobei das Molverhältnis des cyclischen Anhydrids zu dem von der Wasserstoffperoxidquelle bereitgestellten Wasserstoffperoxid 0,8:1 bis 1,3:1 beträgt;
   wobei der oral akzeptable Träger ferner einen oder mehrere Bestandteile umfasst, ausgewählt aus antibakteriellen Mitteln, Mitteln gegen Zahnstein, Feuchthaltemitteln, Tensiden und Kühlmitteln, und
   wobei das cyclische Anhydrid kein cyclisches Anhydrid auf Polymerbasis ist.

**Revendications**

1. Composition de blanchiment pour bain de bouche, comprenant un véhicule acceptable par voie orale, du carbonate de sodium, une source de peroxyde d'hydrogène qui fournit du peroxyde d'hydrogène et un anhydride cyclique qui génère un peracide avec le peroxyde d'hydrogène fourni par la source de peroxyde d'hydrogène.

dans laquelle le rapport molaire entre l'anhydride cyclique et le peroxyde d'hydrogène fourni par la source de peroxyde d'hydrogène est de 0,8:1 à 1,3:1.

dans laquelle le véhicule acceptable par voie orale comprend en outre un ou plusieurs ingrédients choisis parmi les agents antibactériens, les agents antitartre, les humectants, les tensioactifs et les agents rafraîchissants.

dans laquelle l'anhydride cyclique n'est pas un anhydride cyclique à base de polymère.

2. Composition de blanchiment pour bain de bouche selon la revendication 1, dans laquelle la source de peroxyde d'hydrogène comprend au moins un composé parmi le peroxyde d'hydrogène, le peroxyde d'urée, le peroxyde de calcium, le perborate de sodium, un complexe de peroxyde d'hydrogène et de polyvinylpyrrolidone (PVP) et le percarbonate de sodium.

3. Composition de blanchiment pour bain de bouche selon la revendication 1 ou 2, dans laquelle la source de peroxyde d'hydrogène est présente dans la composition de blanchiment en une quantité efficace pour fournir une quantité inférieure ou égale à 2,0 % en poids de peroxyde d'hydrogène.

4. Composition de blanchiment pour bain de bouche selon l'une quelconque des revendications 1 à 3, dans laquelle l'anhydride cyclique est au moins l'un de l'anhydride maléique, l'anhydride succinique, le dianhydride naphtalènetétracarboxylique, l'anhydride phtalique, l'anhydride chloromaléique et l'anhydride dichloromaléique.

5. Composition de blanchiment pour bain de bouche selon l'une quelconque des revendications 1 à 4, dans laquelle l'anhydride cyclique est l'anhydride maléique.

6. Composition de blanchiment pour bain de bouche selon l'une quelconque des revendications 1 à 5, dans laquelle le peracide est le peracide maléique.

7. Composition de blanchiment pour bain de bouche selon l'une quelconque des revendications 1 à 5, dans laquelle l'anhydride cyclique est l'anhydride succinique.

8. Composition de blanchiment pour bain de bouche selon la revendication 7, dans laquelle le peracide est le peracide succinique.

9. Composition de blanchiment pour bain de bouche selon l'une quelconque des revendications 1 à 8, dans laquelle le rapport molaire entre l'anhydride cyclique et le peroxyde d'hydrogène fourni par la source de peroxyde d'hydrogène est d'environ 1:1.

10. Composition de blanchiment pour bain de bouche selon l'une quelconque des revendications 1 à 9, dans laquelle le peracide est généré en trois minutes ou moins après la mise en contact du peroxyde d'hydrogène et de l'anhydride cyclique l'un avec l'autre.

11. Composition de blanchiment pour bain de bouche selon l'une quelconque des revendications 1 à 10, comprenant en outre une source d'ions fluorure.

12. Composition de blanchiment pour bain de bouche selon l'une quelconque des revendications 1 à 11, comprenant en outre un tensioactif.

13. Procédé de blanchiment des dents d'un sujet, comprenant la mise en contact de la composition de blanchiment pour bain de bouche selon l'une quelconque des revendications 1 à 12 avec les surfaces des dents du sujet en ayant besoin.

14. Procédé de génération d'un agent de blanchiment dans une composition de blanchiment pour bain de bouche, comprenant un véhicule acceptable par voie orale, du carbonate de sodium, une source de peroxyde d'hydrogène qui fournit du peroxyde d'hydrogène, et un anhydride cyclique qui génère un peracide avec le peroxyde d'hydrogène fourni par la source de peroxyde d'hydrogène, ledit procédé comprenant la mise en contact de l'anhydride cyclique et du peroxyde d'hydrogène l'un avec l'autre, dans lequel l'agent de blanchiment est un peracide ;

dans lequel le rapport molaire entre l'anhydride cyclique et le peroxyde d'hydrogène fourni par la source de peroxyde d'hydrogène est de 0,8:1 à 1,3:1 ;

dans lequel le véhicule acceptable par voie orale comprend en outre un ou plusieurs ingrédients choisis parmi les agents antibactériens, les agents antitartre, les humectants, les tensioactifs et les agents rafraîchissants, et dans lequel l'anhydride cyclique n'est pas un anhydride cyclique à base de polymère.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2130528 A1
- US 2015196469 A1
- US 3535421 A, Briner
- US 4885155 A, Parran, Jr.
- US 3678154 A, Widder
- US 3959458 A, Agricola
- US 3937807 A, Haefele
- US 4051234 A, Gieske